# EUROPÄISCHE PATENTSCHRIFT

(11) **EP 1 637 067 B1**
(45) Veröffentlichungstag und Bekanntmachung des Hinweises auf die Patenterteilung: **19.11.2008**
(21) Anmeldenummer: 04021588.1
(22) Anmeldetag: 10.09.2004
(51) Int. Cl.: A61B 3/11, G02C 13/00

(54) **Vorrichtung zur Erfassung der Höhe einer Pupillenmitte über dem unteren Rand einer Brillenfassung oder eines Brillenglases**
Device for detecting height of pupil above the bottom edge of a spectacles' frame or of a spectacles' glass
Appareil pour la détection de l'élevation du centre de la pupille vis-à-vis du bord inférieur d'une monture ou d'un verre à lunettes

(43) Veröffentlichungstag der Anmeldung: 22.03.2006
(73) Patentinhaber: Fischer, Eric, 82041 Oberhaching (DE)
(72) Erfinder: Fischer, Eric, 82041 Oberhaching (DE)
(74) Vertreter: Söllner, Udo

(56) Entgegenhaltungen:
- US-A- 5 640 775

## Beschreibung

Die vorliegende Erfindung betrifft eine Vorrichtung zur Erfassung der Höhe einer Pupillenmitte über dem unteren Rand einer Brillenfassung oder eines Brillenglases.

Der Träger oder Benutzer einer Brille hat die Wahl zwischen Brillen mit Brillenfassungen, bei denen die Brillengläser von einer Einfassung aus beispielsweise einer Aluminium- oder Titanlegierung oder einem anderen Werkstoff umfasst sind oder auch rahmenlosen Brillen, bei denen die Brillengläser lediglich von einem Nasensteg und Brillenbügeln fixiert werden. Wenn im vorliegenden Fall von Brillengläsern gesprochen wird, werden darunter die optischen Linsen einer Brille verstanden, die selbstverständlich auch aus einem Kunststoffwerkstoff gefertigt sein können.

Bei den genannten Brillengläsern kann es sich um Einstärkengläser oder auch um Mehrstärkengläser handeln, sogenannte Gleitsichtgläser oder multifokale Linsen oder Gläser. Wenn sich der Benutzer einer solchen Brille im Rahmen einer Neuanschaffung im Optikerfachgeschäft befindet, so müssen zur Anpassung der Brillengläser an den Benutzer verschiedene Zentrierdaten erfasst werden, die bei der Herstellung der Gläser zur Anpassung an die Brillenfassung oder auch die rahmenlose Brille unter Berücksichtigung der spezifischen optometrischen Daten des Benutzers notwendig sind.

Die Hersteller solcher multifokaler Linsen benötigen zur benutzerspezifischen Anpassung der multifokalen Linsen einen Punkt an der Linse, der direkt vor der Pupille liegt, wenn der spätere Benutzer oder Träger der Brille waagerecht geradeaus in Richtung nach vorne sieht.

Der Träger oder Benutzer der spezifisch für ihn anzufertigenden Brille wird also gebeten, bei für ihn normaler und natürlicher Kopfhaltung und Körperhaltung geradeaus zu blicken. Die übliche Vorgehensweise ist dabei derart, dass sich zur Feststellung der Pupillenmitte relativ zum unteren Rand der Brillenfassung - oder bei einer rahmenlosen Brille ― dem unteren Rand des Brillenglases, der Optiker bzw. die Optikerin vor den Benutzer stellt und diesen bittet, den Blick geradeaus in Richtung nach vorne zu richten und gleichzeitig dabei eine normale und natürliche Kopfhaltung und Körperhaltung einzunehmen. Der Optiker bzw. die Optikerin versucht dann, die Pupillenmitte anzuvisieren und mit einem Markierstift eine Markierung an dem Brillenglas ― bzw. bei einer vom Benutzer ausgewählten neuen Brillenfassung, die Demogläser aufweist, am Demoglas anzubringen. Diese bekannte Vorgehensweise weist nun mehrere Nachteile auf.

Einerseits ist es für den Benutzer der für ihn spezifisch anzufertigenden Brille eine unnatürliche Situation, wenn der Optiker bzw. die Optikerin sich vor ihn stellt, was nach vielfach durchgeführten Beobachtungen dazu führt, dass der Benutzer der Brille eine von seiner normalen und natürlichen Kopfhaltung und Körperhaltung abweichende Kopfhaltung und Körperhaltung einnimmt, also beispielsweise deutlich mehr aufrecht steht, als er dies üblicherweise tut und darüber hinaus auch noch den Kopf in Richtung nach hinten neigt.

Diese von der normalen und natürlichen Kopfhaltung und Körperhaltung des Benutzers abweichende Kopfhaltung bzw. Körperhaltung führt zu dem Problem, dass sich ― beispielsweise bei einer Bewegung des Kopfes des Benutzers in Richtung nach hinten ― die Höhe der Pupille relativ zum unteren Rand der Brillenfassung bzw. des Brillenglases ändert.

Wird vom Optiker bzw. der Optikerin die Pupillenmitte dann gemäß dieser unnatürlichen Kopfhaltung und Körperhaltung angenommen und mit dem Markierstift eine entsprechende Markierung am Brillenglas oder dem Demoglas angebracht und auf der Basis dieser Markierung dann der Abstand zum unteren Rand der Brillenfassung oder des Brillenglases gemessen und das Gleitsichtglas dann auf der Basis dieses Messwertes angefertigt, so führt dies zu der schwerwiegenden Folge, dass das gefertigte Gleitsichtglas dem Benutzer nur optimal angepasst ist, wenn dieser später wieder diese unnatürliche Kopfhaltung und Körperhaltung einnimmt. Es liegt in der Natur der Dinge, dass dies zu Problemen bei der Benutzung der neu angefertigten Brille durch den Benutzer führt, sodass die Brille für diesen Benutzer tatsächlich nicht brauchbar ist.

Ein weiteres Problem bei dieser bekannten Vorgehensweise besteht darin, dass sich der Optiker bzw. die Optikerin zur Anbringung der Markierung an dem Brillenglas oder dem Demoglas mit dem Benutzer auf gleicher Augenhöhe befinden muss, um das Problem einer vertikalen Parallaxe zu vermeiden.

Dies wiederum ist nur möglich, wenn der männliche oder weibliche Optiker bzw. im Optikerfachgeschäft beschäftigtes Personal etwa die gleiche Körpergröße besitzt wie der Benutzer. Da dies oftmals nicht der Fall ist, wird der Benutzer gebeten, sich zu setzen, sodass sich die die Messung vornehmende Person vor den Benutzer setzen kann, um in gleicher Augenhöhe die entsprechende Markierung an dem Brillenglas bzw. dem Demoglas anbringen zu können. Selbst dann, wenn in dieser sitzenden Stellung die die Markierung anbringende Person der Meinung ist, dass sie sich mit dem Benutzer in gleicher Augenhöhe befindet, so handelt es sich hierbei um eine subjektive Vorgehensweise, da es keinerlei Bestätigung gibt, dass sich die Augenhöhe des Benutzers mit der Augenhöhe der die Messung vornehmenden Person in Überdeckung befindet.

Entsprechende Beobachtungen haben gezeigt, dass eine Abweichung der Augenhöhen bzw. der Pupillenmittenhöhen von etwa 5 cm üblich ist, was zu dem vorstehend geschilderten Problem der vertikalen Parallaxe führt. Im Ergebnis führt dies zu einer Abweichung des festzustellenden Abstandes zwischen der Pupillenmitte und dem unteren Rand der Brillenfassung oder Brillenglas von etwa 1.1 mm, wobei aber engere Toleranzgrenzen von + / - 0.2 mm gefordert sind.

Auch in dieser sitzenden Position des Benutzers stellt sich vielfach das eingangs geschilderte Problem ein, dass der Benutzer seine Kopfhaltung ändert, was die Messung noch komplizierter macht. Eine geringfügige Veränderung der Kopfhaltung bzw. der Körperhaltung des Benutzers führt nämlich zu einer deutlichen Veränderung der Pupillenmittenhöhe über dem unteren Rand der Brillenfassung bzw. dem Brillenglas.

Zu diesem Problem kommt noch das Problem der vertikalen Parallaxe hinzu, sodass im Ergebnis letztlich die die Messung vornehmenden Personen, also Optiker oder Optikerinnen oder im Fachgeschäft beschäftigtes Personal, eigene subjektive und damit nicht reproduzierbare Vorgehensweisen entwickeln bei der Bestimmung des Abstandes der Pupillenmitte vom unteren Rand des Brillenglases bzw. der Brillenfassung und als Kriterium des Erfolgs der subjektiven Vorgehensweise die Zahl der Kundenbeschwerden verwenden.

Diese Schilderung macht deutlich, dass die Notwendigkeit besteht, diesen Umstand zu beseitigen und damit gleichzeitig auch die Zahl der nicht optimal für den jeweiligen Benutzer gefertigten multifokalen Linsen zu verringern.

Es sind bereits mehrfach Versuche unternommen worden, dieses Problem zu beseitigen. So ist beispielsweise anhand der US 4,505,043 eine zur Anordnung an der Brillenfassung vorgesehene Vorrichtung bekannt geworden. Bei dieser bekannten Vorrichtung handelt es sich um einen Messaufsatz, der an der Brillenfassung angebracht wird und mit dem die Höhe der Pupillenmitte über dem unteren Rand des Brillenglases bzw. der Brillenfassung messbar sein soll. Dieser bekannte Messaufsatz weist aber den Nachteil auf, dass er sich nur für spezifische Fassungsvorneigungswinkel eignet und darüber hinaus auch das Problem der Messung während unnatürlicher Kopf- und Körperhaltung des Benutzers nicht beseitigen kann.

Wie es vorstehend nämlich erläutert wurde, handelt es sich bei der Messung während einer unnatürlichen Kopf- und Körperhaltung des Benutzers um ein schwerwiegendes Problem, welches das Messergebnis deutlich verfälscht. Wenn nun mit diesem bekannten Messaufsatz versucht wird, die Höhe der Pupillenmitte über dem unteren Rand der Brillenfassung bzw. des Brillenglases zu messen, so hat diejenige Person, die diese Messung vornimmt, keine Möglichkeit zu verifizieren, ob der Benutzer der spezifisch für ihn anzufertigenden Brille eine Kopf- bzw. Körperhaltung einnimmt, die von seiner normalen und natürlichen Kopf- bzw. Körperhaltung beim Blick in die Ferne abweicht.

Eine weitere Messvorrichtung ist anhand der US 5,640,219 bekannt geworden. Bei dieser Messvorrichtung wird der Kopf des Benutzers zur Feststellung der Höhe der Pupillenmitte in einem Rahmen fixiert, was - ohne dass weitere Ausführungen hierzu erforderlich erscheinen - einer natürlichen Kopf- und Körperhaltung während der Messung zuwiderläuft. Darüber hinaus handelt sich bei dieser bekannten Vorrichtung um ein ausgesprochen komplexes und aufwändiges und damit teures Gerät.

Anhand der US 5,640,775 ist eine Vorrichtung bekannt geworden, mit der an einer multifokalen Linse der Übergangsbereich zwischen progressiven Stärken des Brillenglases in vertikaler Richtung festgelegt werden kann. Dazu besitzt die Vorrichtung einen schwenkbaren Markierbügel, mit dem an der Aussenseite der Linse auf Höhe der Nullblickrichtung des Benutzers der Brille eine optische Markierung angebracht werden kann, die den Übergangsbereich bestimmt.

Ausgehend hiervon liegt der vorliegenden Erfindung nunmehr die Aufgabe zugrunde, eine Vorrichtung zu schaffen, mittels der die Höhe der Pupillenmitte über dem unteren Rand einer Brillenfassung oder eines Brillenglases gemessen werden kann, die sich bei einer natürlichen Haltung des Kopfes und des Körpers des Benutzers auch tatsächlich einstellt.

Die zur Lösung dieser Aufgabe geschaffene Vorrichtung weist die Merkmale nach dem Anspruch 1 auf. Vorteilhafte Ausgestaltungen hiervon sind in den weiteren Ansprüchen beschrieben.

Die nach der Erfindung vorgesehene Vorrichtung, die im folgenden auch als Messaufsatz bezeichnet wird, ist zur Anordnung an der Brillenfassung ausgebildet, die sich der Benutzer beispielsweise bei der Neuanschaffung der Brille ausgesucht hat. Bei einer rahmenlosen Brille kann der erfindungsgemäße Messaufsatz an den Gläsern aufgesetzt werden. Der Benutzer trägt also die ausgewählte Brillenfassung, an der der Messaufsatz nach der Erfindung angeordnet ist. Der Benutzer kann nun beispielsweise gebeten werden, seinen Blick auf einen Punkt in der Ferne zu richten, er kann dabei im Optikerfachgeschäft herumgehen, oder auch eine sitzende Position einnehmen, beispielsweise eine Fernsehsendung beobachten, oder dergleichen, in jedem Fall eine natürliche und ungestörte Kopfhaltung bzw. Körperhaltung einnehmen, also beispielsweise den Kopf in Richtung nach vorne neigen oder den Kopf in Richtung nach hinten neigen, je nachdem welche Kopfhaltung bzw. Körperhaltung der Benutzer beim Blick waagrecht geradeaus nach vorne üblicherweise einnimmt.

Die am Messaufsatz nach der Erfindung vorgesehene erste Einrichtung ist an einer weitgehend horizontalen Drehachse drehbar und reagiert auf die sich einstellende natürliche Kopfhaltung und Körperhaltung des Benutzers durch eine Drehbewegung und ermöglicht die Bestimmung der benutzerspezifischen Nullblickrichtung. Diese erste Einrichtung ist dabei so ausgebildet, dass sich die bei natürlicher Kopf- und Körperhaltung des Benutzers einstellende Nullblickrichtung festgehalten, gleichsam eingefroren werden kann, wobei auf der Basis dieser benutzerspezifischen Nullblickrichtung dann mittels der zweiten Einrichtung die Pupillenmitte des Benutzers ermittelt werden kann.

Bei der nach der Erfindung vorgesehenen ersten Einrichtung handelt es sich um ein massebehaftetes Schwenkbauteil , welches an der horizontalen Drehachse frei drehbar gelagert ist derart, dass eine durch zwei an dem Schwenkbauteil vorgesehene Bezugstrecken aufgespannte Ebene bei veränderlicher Kopf- und Körperhaltung des Benutzers parallel zur benutzerspezifischen Nullblickrichtung verläuft. Es wird damit mit anderen Worten mittels des frei drehbar gelagerten Schwenkbauteils bzw. zweier daran vorgesehener Bezugsstrecken eine Ebene aufgespannt, die bei sich verändernder Kopf- und Körperhaltung des Benutzers der benutzerspezifischen Nullblickrichtung folgt, also eine Ebene aufgespannt wird, die der horizontalen Blickrichtung des Benutzers folgt. Wenn nun der Benutzer beispielsweise in einer für ihn angenehmen und damit natürlichen Kopf- und Körperhaltung beim Blick in die Ferne herumgeht, so verläuft die durch die Bezugsstrecken aufgespannte Ebene in einem parallelen Abstand, wobei der Abstand auch den Wert Null annehmen kann, entlang der horizontalen Blickrichtung des Benutzers. Die durch die beiden Bezugsstrecken aufgespannte Ebene entspricht daher in jedem betrachteten Augenblick der benutzerspezifischen Nullblickrichtung.

Dadurch, dass nach einer Weiterbildung der Erfindung das Schwenkbauteil arretierbar ausgebildet ist, dass nämlich in der während natürlicher Kopf- und Körperhaltung des Benutzers arretierten Stellung die von den Bezugsstrecken aufgespannte Ebene parallel zur benutzerspezifischen Nullblickrichtung verläuft, kann diese benutzerspezifische Nullblickrichtung durch eine Arretierung des Schwenkbauteils festgehalten, gleichsam eingefroren werden.

Das Schwenkbauteil wird beispielsweise vom Optiker oder Bedienpersonal im Fachgeschäft in einem beliebigen Augenblick natürlicher Kopf- und Körperhaltung des Benutzers arretiert, sodass die von den Bezugsstrecken aufgespannte Ebene der benutzerspezifischen Nullblickrichtung eindeutig entspricht.

Die zweite Einrichtung sind zwei an der ersten Einrichtung im Abstand angeordnete und eine vorbestimmte Länge aufweisende Stifte, die gleichzeitig die Bezugsstrecken sind. Die beiden am Schwenkbauteil vorgesehenen Stifte spannen also die Ebene auf, die der benutzerspezifischen Nullblickrichtung bei natürlicher Kopf- und Körperhaltung des Benutzers entspricht.

Nachdem diese benutzerspezifische Nullblickrichtung während einer natürlichen Kopf- und Körperhaltung des Benutzers festgestellt, gleichsam eingefroren worden ist, verändert sich diese benutzerspezifische Nullblickrichtung durch eine spätere Veränderung der Kopf- und Körperhaltung des Benutzers nicht mehr.

Die beiden, eine vorbestimmte Länge aufweisenden Stifte bilden gleichzeitig eine Sichtebene aus, entlang derer die Bedienperson überprüfen kann, ob sich die Höhe der Pupillenmitte der Bedienperson in gleicher Höhe mit der Höhe der Pupillenmitte des Benutzers befindet.

Zu diesem Zweck kann sich der Benutzer beispielsweise in eine sitzende Stellung begeben und die Bedienperson setzt sich dem Benutzer gegenüber und richtet ihren Blick entlang der durch die beiden Stifte aufgespannte Ebene, d. h. also entlang der benutzerspezifischen Nullblickrichtung auf die Pupillenmitte des Benutzers, so dass visueller Kontakt zwischen der Pupillenmitte des Benutzers und der Pupillenmitte der Bedienperson besteht. Damit wird gleichzeitig das Problem der vertikalen Parallaxe vermieden.

Um es dem Bedienpersonal schnell und einfach zu ermöglichen festzustellen, ob sich die Pupillenmitte der Bedienperson in Höhe der benutzerspezifischen Nullblickrichtung befindet, können die beiden am Schwenkbauteil angebrachten Stifte beispielsweise verschiedenfarbig ausgebildet sein, sodass der von der Bedienperson aus betrachtete in Richtung zum Benutzer näher liegende hinten liegende Stift eine andere Farbe besitzen kann als der näher in Richtung zur Bedienperson liegende Stift, sodass die Bedienperson die gleiche Pupillenmittenhöhe dadurch verifizieren kann, dass sie sich dem Benutzer beispielsweise gegenüber stellt oder setzt und solange die Steh- oder Sitzhöhe modifiziert, bis der anders farbige hinten liegende Stift vollständig von dem vorne liegenden Stift abgedeckt wird. Dies ist gleichzeitig der Augenblick, zu dem sich die Pupillenmitte des Benutzers mit der Pupillenmitte der Bedienperson deckt. In diesem Augenblick kann die Bedienperson beispielsweise mit einem Markierstift einen farbigen Markierpunkt an dem Brillenglas oder dem Demoglas exakt in der Höhe der Pupillenmitte des Benutzers anbringen. Mittels einer einfachen Messvorrichtung in der Form eines Messlineals oder dergleichen kann die Bedienperson sodann den Abstand zwischen dem unteren Rand der Brillenfassung oder dem Brillenglas oder der Pupillenmitte messen. Damit ist das Problem einer Fehlmessung aufgrund einer unnatürlichen Kopf- und Körperhaltung des Benutzers und der vertikalen Parallaxe vermieden.

Wie es vorstehend bereits ausgeführt wurde, ist der Messaufsatz nach der Erfindung zur Anordnung am oberen Rand der Brillenfassung bzw. oberen Rand von Brillengläsern bei einer rahmenlosen Brille vorgesehen. Das in der nicht arretierten Stellung frei drehbare horizontal angelenkte Schwenkbauteil weist eine vorbestimmte Längserstreckung auf, die sich nicht notwendigerweise bis in den Bereich der Augenmitte des Benutzers erstreckt, um eine natürliche Kopf- und Körperhaltung des Benutzers nicht zu stören oder zu behindern.

Wenn sich nun die Pupillenmitte der Bedienperson in Höhe der durch die beiden Stifte aufgespannten Bezugsebene befindet, so befindet sich die Pupillenmitte der Bedienperson etwas oberhalb der benutzerspezifischen Nullblickrichtung. Beobachtungen bei in der Praxis durchgeführten Bestimmungen der Höhe der Pupillenmitte des Benutzers über dem unteren Rand einer Brillenfassung oder eines Brillenglases haben ergeben, dass der Abstand zwischen dem Benutzer und der Bedienperson üblicherweise im Bereich von etwa 60 cm liegt. Befindet sich nun die Pupillenmitte der Bedienperson in einer geringfügigen Höhendifferenz außerhalb der benutzerspezifischen Nullblickrichtung, so führt dies bei dem geschildertem Abstand von 60 cm zu einem Messfehler von 0.15 mm, also einem Messfehler, der bereits innerhalb der von den Herstellern der Brillengläsern geforderten Messtoleranz liegt.

Um aber auch dieser Gegebenheit Rechnung zu tragen, ist es nach einer Weiterbildung der Erfindung vorgesehen, dass die Stifte querschnittlich quaderförmig ausgebildet sind und Sichtkanten ausbilden, wobei sich eine durch vorbestimmte Sichtkanten aufgespannte Ebene in einem vorbestimmten Abstand zum Brillenglas mit einer die Pupillenmitte und die benutzerspezifische Nullblickrichtung enthaltenden Nullblickrichtungsebene derart schneidet, dass sich eine in Höhe der Schnittgerade befindende Pupillenmitte eines Betrachters genau in der Höhe der Pupillenmitte des Benutzers befindet.

Nach dieser Ausführungsform werden mit anderen Worten die am Schwenkbauteil vorgesehenen Stifte nicht querschnittlich rund, sondern querschnittlich quaderförmig ausgebildet, sodass die Ecken der Quader Sichtkanten ausbilden. Bei der sich an die Erfassung der benutzerspezifischen Nullblickrichtung anschließenden Messung setzt sich nun eine Bedienperson im Abstand von beispielsweise 60 cm dem Benutzer gegenüber.

Die Bedienperson visiert nun die obere vordere Kante des zu ihr näher liegenden quaderförmigen Stifts an und bringt diese Sichtkante in Überdeckung mit der oberen vorderen Kante des näher zum Benutzer liegenden zweiten quaderförmigen Stifts, der eine geringfügig größere Höhe besitzt als der weiter vorne in Richtung zur Bedienperson liegende erste quaderförmige Stift. Durch diese beiden Sichtkanten wird nun eine von der Bedienperson einfach anzuvisierende Ebene aufgespannt, die sich im Abstand von beispielsweise 60 cm zum Benutzer mit der die benutzerspezifische Nullblickrichtung enthaltenden Nullblickrichtungsebene schneidet, sodass sich die aus den beiden fiktiven genannten Ebenen ergebene Schnittgerade exakt in Höhe der Pupillenmitte des Benutzers befindet. Damit liegt die Pupillenmitte des Betrachters, also beispielsweise des Optikers oder einer Bedienperson im Optikerfachgeschäft exakt auf der Höhe der Pupillenmitte des Benutzers.

Bei dem nach der Erfindung vorgesehenen Messaufsatz handelt es sich darüber hinaus um eine ausgesprochen kostengünstig herzustellende Vorrichtung, die nur wenige Bauteile aufweist. Der Messaufsatz weist nach einer Weiterbildung der Erfindung eine langgestreckten Träger auf, an dessen Enden jeweils zwei weitgehend vertikal verlaufende Bügel angeordnet sind, mittels derer die Vorrichtung lösbar an der Brillenfassung oder an dem Brillenglas anbringbar ist und an dessen axialer Mitte ein gabelförmiger Ausleger angeordnet ist, an dem das Schwenkbauteil drehbar angeordnet ist. An dem Ausleger kann ein Arretierbügel verschwenkbar angelenkt sein derart, dass mittels einer Verschwenkbewegung des Arretierbügels das Schwenkbauteil an seiner frei drehbaren Stellung in die arretierte Stellung überführt werden kann.

Wenn sich somit der Arretierbügel relativ zum Schwenkbauteil in einer offenen Stellung befindet, so folgt das Schwenkbauteil aufgrund seiner frei drehbaren Anordnung am Ausleger den Kopf- und Körperbewegungen des Benutzers durch Drehung. In einem Augenblick natürlicher Kopf- und Körperhaltung des Benutzers kann der Arretierbügel aus seiner offenen Stellung in eine Stellung mit Kontakt mit dem Schwenkbauteil verschwenkt werden, sodass er am Schwenkbauteil anliegt und das Schwenkbauteil fixiert.

Vorstehend wurde bereits ausgeführt, dass die Bedienperson beispielsweise mittels eines Markierstifts eine Markierung am Brillenglas oder dem Demoglas der vom Benutzer ausgewählten Brillenfassung anbringen kann und dann mittels eines Messlineals den Abstand zwischen dem die Pupillenmitte darstellenden Bezugspunkt und dem unteren Rand der Brillenfassung oder dem Brillenglas misst.

Nach einer Weiterbildung der Erfindung kann an einem vertikal verlaufenden Bügel eine skalierte Messeinrichtung verschiebbar angebracht werden derart, dass der Nullpunkt der Messeinrichtung mit dem unteren Rand der Brillenfassung oder dem Brillenglas in Überdeckung gebracht werden kann und der Abstand vom Nullpunkt zur Pupillenmitte mittels der Messeinrichtung direkt bestimmt, d. h. abgelesen werden kann. Zu diesem Zweck setzt sich die Bedienperson dem Benutzer beispielsweise gegenüber und misst ― nachdem sie ihre Pupillenmitte in Überdeckung gebracht hat mit der Pupillenmitte des Benutzers ― den Abstandswert vom unteren Rand des Brillenglases oder Brillenfassung direkt von der Messeinrichtung ab.

Nach einer Weiterbildung der Erfindung kann an dem Schwenkbauteil eine skalierte Messeinrichtung vorgesehen sein, mittels der die Fassungsvorneigung der Brillenfassung direkt am Schwenkbauteil abgelesen werden kann.

Nach der bisherigen Schilderung wird der Abstand zwischen der Pupillenmitte des Benutzers und dem unteren Rand der Brillenfassung bzw. des Brillenglases manuell bestimmt. Nach einer Weiterbildung der Erfindung können an dem langgestreckten Träger des Messaufsatzes zwei Referenzpunkte in einem vorbestimmte Abstand angeordnet werden, die eine Referenzskala bilden, mittels der Abmessungen aus einer von dem Benutzer mit Brillenfassung und Messaufsatz aufgenommenen Abbildung mittels elektronischer Bildverarbeitung in skalierte Werte konvertierbar sind und die Höhe der Pupillenmitte über dem unteren Rand der Brillenfassung oder des Brillenglases bestimmt werden kann.

Nach dieser Weiterbildung des erfindungsgemäßen Messaufsatzes sind beispielsweise an den beiden distalen Enden des langgestreckten Trägers zwei Referenzpunkte in einem vorbestimmten Abstand vorgesehen, sodass mittels elektronischer Bildverarbeitung alle an einer vom Benutzer mit Brillenfassung und Vorrichtung aufgenommenen Abbildung vorhandene Abmessungen auf den Abstand zwischen den beiden Referenzpunkten, der Referenzskala, referenziert werden können und somit in diskrete Werte, beispielsweise Millimeterwerte, umgerechnet werden können. Damit kann beispielsweise der Abstand der Pupillenmitte des Benutzers vom unteren Rand der Brillenfassung oder des Brillenglases mittels elektronischer Bildverarbeitung bestimmt werden.

Auch kann nach dieser Ausführungsform eine Abbildung von dem Benutzer mit der Brillenfassung und dem Messaufsatz aufgenommen werden und beispielsweise ein Ausdruck hiervon erstellt werden, an dem die Höhe der Pupillenmitte über dem unteren Rand der Brillenfassung oder des Brillenglases unter Berücksichtigung der Referenzskala gemessen werden kann oder auch eine entsprechende Messung an einem vorzugsweise planen Bildschirm durchgeführt werden.

Schließlich ist es nach einer Weiterbildung der Erfindung auch noch vorgesehen, dass an den beiden Gabeln des gabelförmigen Auslegers jeweils eine vertikale Linie in einer vorbestimmten Relativstellung zu den Referenzpunkten angebracht ist derart, dass zur Vermeidung einer vertikalen Parallaxe mittels elektronischer Bildverarbeitung bestimmbar ist, ob die aufgenommene Abbildung vertikal von vorne oder in einer von der Vertikalen seitlich abweichenden Richtung aufgenommen wurde. Dadurch ist es möglich, den Benutzer mit der Brillenfassung und dem Messaufsatz beispielsweise mittels einer mit einer elektronischen Datenverarbeitungsvorrichtung verbundenen Kamera aufzunehmen und die Höhe der Pupillenmitte mittels elektronischer Bildverarbeitung zu bestimmen, wie dies vorstehend bereits angeführt wurde. Mittels der beiden vertikalen Linien an den Gabeln des gabelförmigen Auslegers kann dann festgestellt werden, ob sich der Benutzer und damit der Messaufsatz im Augenblick der Aufnahme der Abbildung direkt vertikal vor der Kamera befunden hat oder die Abbildung von der Seite aufgenommen wurde, sodass im Rahmen der elektronischen Bildverarbeitung für den Winkelversatz eine Kompensation getroffen wird.

Die Erfindung wird nachfolgend anhand der Zeichnung näher erläutert. Diese zeigt in:
Fig. 1 eine perspektivische Ansicht auf eine Ausführungsform eines Messaufsatzes nach der vorliegenden Erfindung;

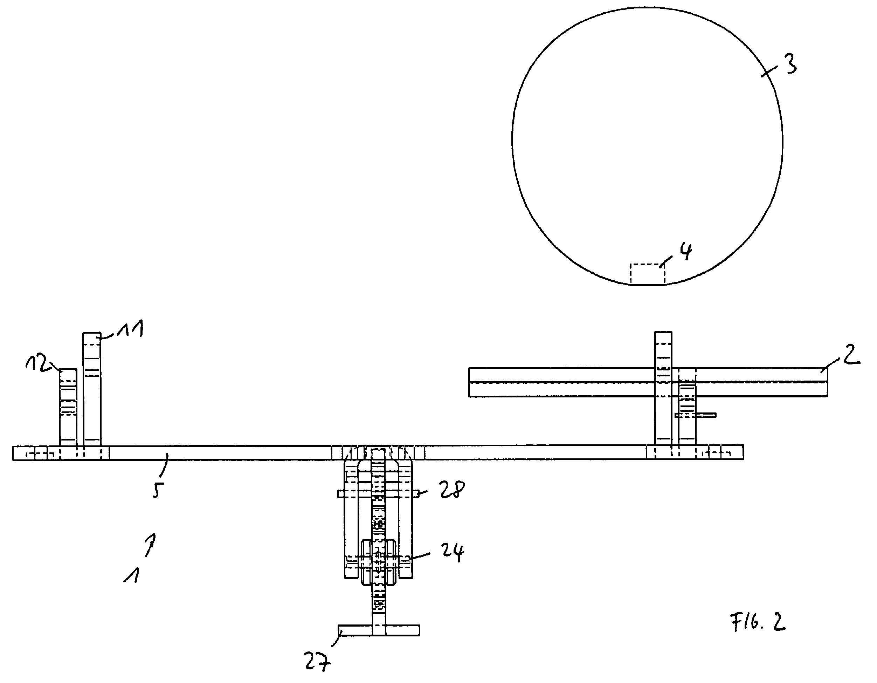

Fig. 2 eine Ansicht auf den Messaufsatz nach Fig. 1 von oben;
Fig. 3 eine Seitenansicht auf den Messaufsatz;
Fig. 4 eine Vorderseitenansicht auf den Messaufsatz; und
Fig. 5 eine schematische Darstellung zur Funktionsweise des Messaufsatzes.

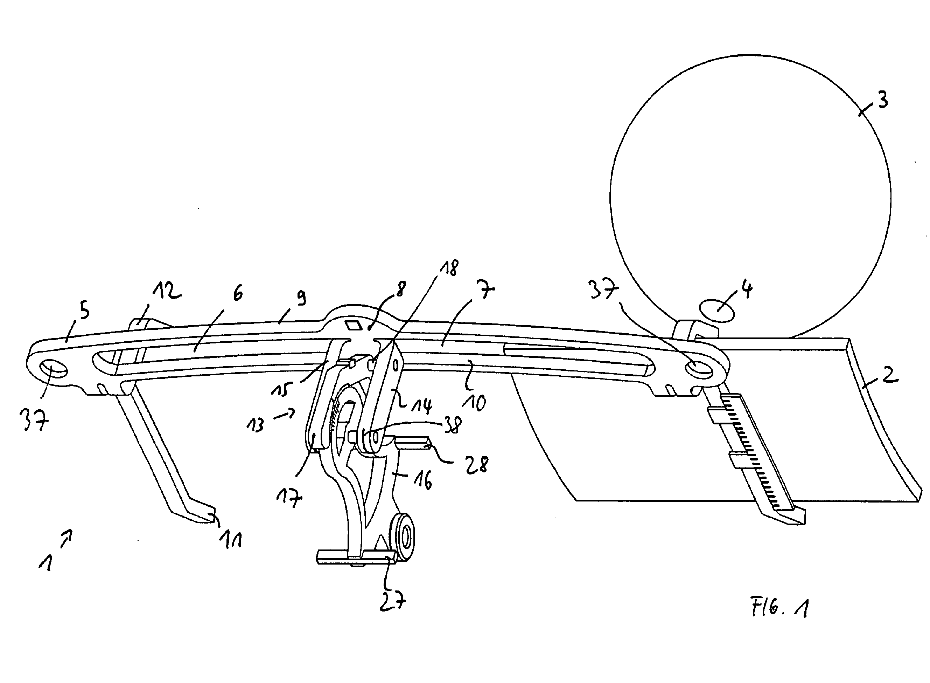

Fig. 1 der Zeichnung zeigt in einer perspektivischen Darstellung von schräg oben eine im Weiteren als Messaufsatz 1 bezeichnete Ausführungsform einer Vorrichtung nach der Erfindung. Mit dem Bezugszeichen 2 wird ein Brillenglas bezeichnet, das Bezugszeichen 3 bezeichnet ein schematisch dargestelltes Auge des Benutzers und das Bezugszeichen 4 seine Pupille.

Wie es ohne weiteres anhand der Zeichnung ersichtlich ist, weist der Messaufsatz 1 einen langgestreckten Träger 5 auf, der eine insgesamt langgestreckte kreisbogensegmentförmige Konfiguration besitzt. Der Träger kann aus Gewichtsersparnisgründen beispielsweise aus einer Aluminiumlegierung oder einem Kunststoffwerkstoff gefertigt sein und besitzt zwei Ausnehmungen 6, 7, die ebenfalls der Gewichtsersparnis dienen.

Im Bereich zwischen den beiden Ausnehmungen 6, 7 weist der Träger 5 einen Steg 8 auf, der den Oberzug 9 mit dem Unterzug 10 des Trägers 5 verbindet.

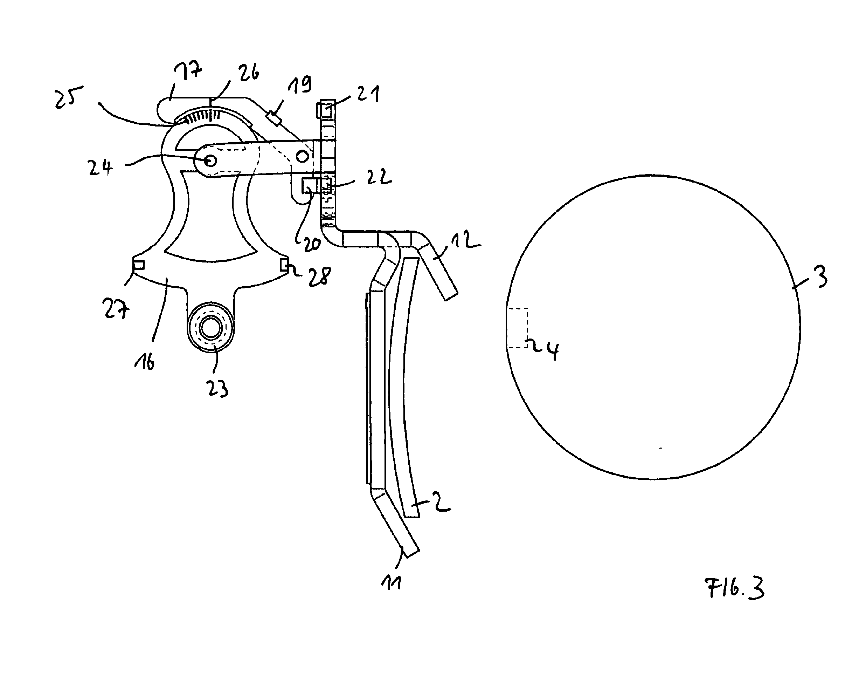

Im Bereich der beiden distalen Enden des Trägers 5 sind jeweils zwei sich vertikal erstreckende Bügel 11, 12 angeordnet, die eine voneinander abweichende Länge aufweisen und so ausgebildet sind, dass sie zwischen sich das gekrümmt ausgebildete Brillenglas 2 aufnehmen können, wobei sich dies beispielsweise anhand von Fig. 3 der Zeichnung ergibt.

Mit den Bügeln 11, 12 kann der Messaufsatz 1 auf ein nicht dargestelltes Brillengestell von oben aufgesetzt werden, sodass das Brillengestell bzw. die Brillengläser 2 zwischen den an beiden distalen Enden des Messaufsatzes 1 vorgesehenen Bügeln 11, 12 aufgenommen werden können und der Messaufsatz 1 am Brillengestell bzw. bei einer rahmenlosen Brille an den beiden Gläsern 2 rutschfest aufliegt.

Der Steg 8 des Trägers 5 wird von einem sich vom Träger 5 im rechten Winkel in Richtung nach vorne gerichteten Ausleger 13 so umfasst, dass sich ein Zwischenraum zwischen zwei gobelförmigen Armen 14, 15 ausbildet der zur Aufnahme eines massebehafteten Schwenkbauteils 16 vorgesehen ist.

In der Zeichnung wird das Schwenkbauteil 16 in einer von einem Arretierbügel 17 arretierten Stellung gezeigt, in der der an einer weitgehend horizontal verlaufenden Schwenkachse 18 verschwenkbare Arretierbügel 17 an dem in einer Draufsichtansicht (siehe Fig. 3) weitgehend glockenförmig ausgebildeten Schwenkbauteil 16 aufliegt.

Der Arretierbügel 17 weist an seiner dem Steg 8 bzw. dem Träger 5 zugewandten Rückseite kleine Permanentmagnete 19, 20 auf, mittels deren er mit am Steg 8 vorgesehenen Permanentmagneten 21, 22 in der in den Zeichnungen dargestellten arretierten Stellung bzw. beim Kontakt des Permanentmagnets 19 mit dem Permanentmagnet 21 in einer offenen Stellung arretiert werden kann, in der er sich aus der mit dem Schwenkbauteil 16 in Kontakt befindenden Stellung gelöst ist, sodass das über einen massebehafteten Körper 23 massebehaftete Schwenkbauteil 16 an einer weitgehend horizontal verlaufenden Drehachse 24 verdrehbar ist.

Wie es beispielsweise anhand von Fig. 3 der Zeichnung ersichtlich ist, weist das Schwenkbauteil 16 an seiner dem Arretierbügel 17 zugewandten Seite eine Messeinrichtung 25 auf, mittels der an einer am Arretierbügel 17 vorgesehenen Markierung der Fassungsvorneigungswinkel der Brillenfassung erfasst werden kann.

An dem glockenförmig ausgebildeten Schwenkbauteil 16 sind zwei im Querschnitt quaderförmige und eine vorbestimmte Längserstreckung aufweisende Ausrichtstifte 27, 28 vorgesehen, wobei der näher am Träger 5 liegende Ausrichtstift 28 eine größere Höhe besitzt als der weiter vom Träger 5 abseits liegende Stift 27.

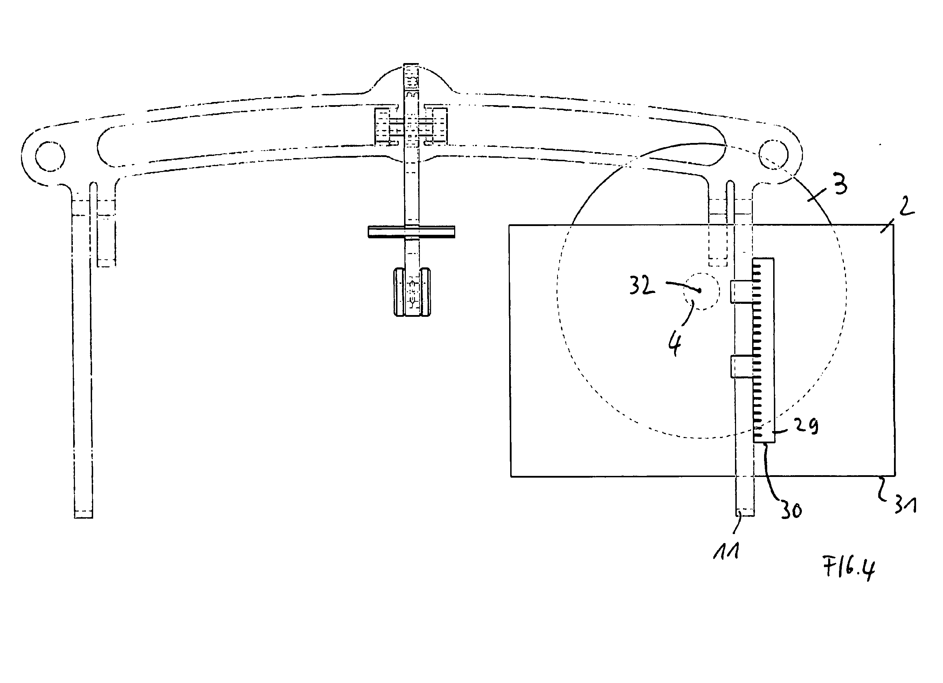

An dem beispielsweise in Fig. 4 der Zeichnung ersichtlichen Bügel 11 ist eine in Axiallängsrichtung des Bügels 11 verschiebbare Messeinrichtung 29 vorgesehen, die so an dem Bügel 11 verschoben werden kann, dass der Nullpunkt 30 der Messeinrichtung 29 mit dem unteren Rand 31 des Brillenglases 2 in Überdeckung gebracht werden kann und der Abstand vom unteren Rand 31 zur Pupillenmitte 32 der Pupille 4 an der Messeinrichtung 29 direkt abgelesen werden kann.

Zur Messung des Abstandes vom unteren Rand 31 des Brillenglases 2 zur Pupillenmitte 32 wird der Messaufsatz 1 mittels der Bügel 11, 12 am Brillengestell aufgesetzt. Der Arretierbügel 17 wird in seine aus dem Kontakt mit dem Schwenkbauteil 16 gelöste Stellung bewegt, indem er nach oben geklappt wird, sodass die beiden Magnete 20, 22 außer Kontakt gelangen und der Arretierbügel 17 mittels des Permanentmagnets 19 und des Permanentmagnets 21 am Trägerkörper 5 bzw. dem Steg 8 gehalten wird.

Der das Brillengestell und den Messaufsatz 1 tragende Benutzer wird gebeten, eine natürliche Kopf- und Körperhaltung einzunehmen und dabei einen in der Ferne lie genden Punkt zu betrachten. Der Benutzer kann dabei im Fachgeschäft stehen, herum gehen, oder sich unterschiedliche Punkte in der Ferne betrachten, sodass sich seine für ihn spezifische eigene natürliche Kopfhaltung bzw. Körperhaltung einstellt, die sich auch dann einstellt, wenn er später die für ihn spezifisch gefertigte Brille tragen wird.

Neben dieser geschilderte Situation kann der Benutzer auch eine andere Stellung einnehmen, wie beispielsweise eine sitzende Stellung im Auto, am Schreibtisch oder vor dem Computermonitor, sodass unter zur Hilfenahme des erfindungsgemäßen Messaufsatzes auch anwendungsfallspezifische Brillen möglich sind, die der beim jeweils anwendungsspezifischen Fall eingenommenen Kopf- und Körperhaltung des Benutzers Rechnung tragen.

Das Schwenkbauteil folgt den Bewegungen des Kopfes bzw. des Körpers des Benutzers nicht nach, sondern dreht sich an der Drehachse 24 relativ zum Träger 5, sodass die von den Ausrichtstiften 27, 28 aufgespannte fiktive Ebene der benutzerspezifischen horizontalen Sichtlinie, also der benutzerspezifischen und auch anwendungsfallspezifischen Nullblickrichtung entspricht.

Da der Benutzer bei dieser Erfassung der benutzerspezifischen Nullblickrichtung nicht durch eine beispielsweise vor ihm stehende Bedienperson gestört wird, nimmt er eine natürliche Kopf- und Körperhaltung ein, sodass die von den Ausrichtstiften 27, 28 aufgespannte Ebene der benutzerspezifischen Nullblickrichtung entspricht.

Diese natürliche Kopf- und Körperhaltung des Benutzers kann vom Optiker bzw. der Bedienperson festgestellt und beispielsweise im Rahmen eines Kundengespräches mit dem Benutzer verifiziert werden, sodass der Optiker bzw. die Bedienperson während der natürlichen Kopf- und Körperhaltung des Benutzers den Arretierbügel 17 umlegt ― wobei der Optiker zu diesem Zweck an den Benutzer beispielsweise von der Seite aus oder von hinten herantritt, so dass der Benutzer in seiner natürlichen Kopf-und Körperhaltung nicht gestört wird - sodass der Permanentmagnet 20 mit dem Permanentmagneten 22 in Kontakt kommt und die benutzerspezifische Nullblickrichtung durch den Kontakt des Arretierbügels 17 mit dem Schwenkbauteil 16 festgehalten wird, d. h. eingefroren wird.

Die Erfassung der Höhe der Pupillenmitte 32 über dem unteren Rand 31 des Brillenglases 2 erfolgt dann auf der Basis dieser so ermittelten benutzerspezifischen Nullblickrichtung. Zu diesem Zweck kann der Optiker bzw. die Bedienperson unabhängig von der Stellung des Benutzers bei der Ermittlung der benutzerspezifischen Nullblickrichtung den Benutzer beispielsweise bitten, sich zu setzen und dann eine dem Benutzer gegenübersitzende Stellung in einem Abstand von etwa 60 cm einnehmen. Hierbei verändert sich die benutzerspezifische Nullblickrichtung nicht mehr, da sich der Arretierbügel 17 mit dem Schwenkbauteil 16 in Kontakt befindet und eine weitere Lageveränderung des Schwenkbauteils 16 nicht mehr möglich ist.

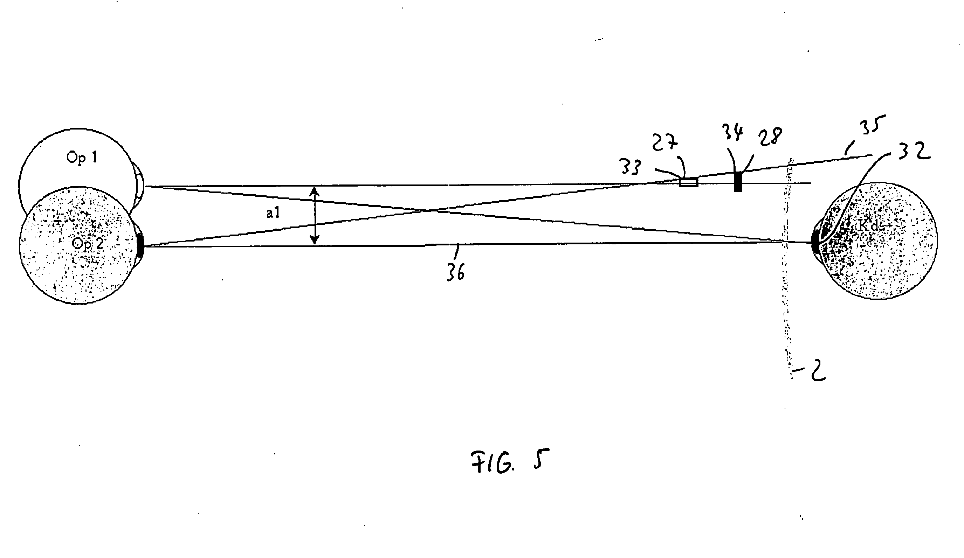

Der Benutzer Kd nach Fig. 5 sitzt der Bedienperson bzw. dem Optiker Op 1 im Abstand von etwa 60 cm gegenüber und der Optiker Op 1 bringt den hinteren Ausrichtstift 28 mit dem vorderen Ausrichtstift 27 optisch in Überdeckung und kann an dem Brillenglas 2 beispielsweise mittels eines Markierstifts eine Markierung anbringen, die die Pupillenmitte des Benutzers Kd wiedergibt, wobei während dieses Vorgangs der Benutzer Kd dem Optiker direkt in die Pupillenmitte blickt.

Damit ist bereits die von den Herstellern von multifokalen Gläsern geforderte Genauigkeit der Höhe der Pupillenmitte über dem unteren Rand des Glases bzw. der Brillenfassung erreichbar.

Wie es anhand von Fig. 5 der Zeichnung ersichtlich ist, liegen die Ausrichtstifte 27, 28 aber nicht in Höhe der Pupillenmitte 32, sondern im Abstand a1 darüber. Trotz dieses Abstandes kann aber mit dem erfindungsgemäßen Messaufsatz 1 bereits die von den Herstellern der Gleitsichtgläser geforderte Genauigkeit erreicht werden.

Bei der in der Zeichnung dargestellten Ausführungsform sind die Ausrichtstifte 27, 28 querschnittlich quaderförmig ausgebildet, wobei der hintere Ausrichtstift 28 eine um den Betrag von etwa 0.175 mm größere Höhe aufweist als der vordere Ausrichtstift 27.

Damit wird es dem Optiker möglich, über die vordere obere Sichtkante 33 des vorderen Ausrichtstiftes 27 und die vordere obere Sichtkante 34 des hinteren Ausrichtstiftes 28 eine Referenzsichtebene 35 zu bilden, die sich mit der die benutzerspezifische Nullblickrichtung enthaltende Nullblickrichtungsebene 36 in Abstand von etwa 60 cm vom Brillenglas 2 mit der Mitte der Pupille des Optikers schneidet, sodass sich die Pupillenmitte Op 2 des Optikers exakt auf der Höhe der Pupillenmitte 32 des Benutzers Kd befindet.

Der nach der Erfindung vorgesehene Messaufsatz ermöglicht es, die Höhe der Pupillenmitte über dem unteren Rand der Brillenfassung oder dem Brillenglas in reproduzierbarer Weise bei natürlicher Kopf- und Körperhaltung des Benutzers zu ermitteln. Zudem wird das Problem der vertikalen Parallaxe aufgrund unterschiedlicher Augenhöhe des Benutzers und des Optikers bzw. einer Bedienperson vermieden.

Mittels am Träger 5 vorgesehener zweier Referenzpunkte 37, die im vorbestimmten Abstand zueinander an den distalen Enden des Trägers 5 angeordnet sind, ist eine Referenzskala geschaffen, anhand der mittels elektronischer Bildverarbeitung der Abstand zwischen dem unteren Rand 31 und der Pupillenmitte 32 errechnet werden kann auf der Basis einer Abbildung, die den Benutzer mit Brillenfassung und Messaufsatz 1 enthält.

Zu diesem Zweck sind an den Armen 14, 15 auch vertikale Linien 38 vorgesehen, die im vorbestimmten Abstand zu den Referenzpunkten 37 vorgesehen sind und anhand deren Relativlage zu den Referenzpunkten 37 bestimmt werden kann, ob die Abbildung vertikal von vorne oder im Winkel zur Vertikalenebene aufgenommen worden ist.

Obwohl vorstehend die Höhe der Pupillenmitte über dem unteren Rand einer Brillenfassung oder eines Brillenglases ermittelt wurde, ist es nach der Erfindung auch möglich, den Abstand der Pupillenmitte zu einem beliebigen, am Brillenglas oder der Brillenfassung vorhandenen oder errechneten Referenzpunkt zu bestimmen, also beispielsweise zu dem errechneten Mittelpunkt des Brillenglases.

Hinsichtlich vorstehend im Einzelnen nicht näher erläuterter Merkmale der Erfindung wird im Übrigen ausdrücklich auf die Ansprüche und die Zeichnung verwiesen.

### Bezugszeichenliste

- 1: Messaufsatz
- 2: Brillenglas
- 3: Auge
- 4: Pupillenmitte
- 5: Träger
- 6: Ausnehmung
- 7: Ausnehmung
- 8: Steg
- 9: Oberzug
- 10: Unterzug
- 11: Bügel
- 12: Bügel
- 13: Ausleger
- 14: Arm
- 15: Arm
- 16: Schwenkbauteil
- 17: Arretierbügel
- 18: Schwenkachse
- 19: Permanentmagnet
- 20: Permanentmagnet
- 21: Permanentmagnet
- 22: Permanentmagnet
- 23: Massekörper
- 24: Drehachse
- 25: Messeinrichtung
- 26: Markierung
- 27: Ausrichtstift
- 28: Ausrichtstift
- 29: Messeinrichtung
- 30: Nullpunkt
- 31: unterer Rand
- 32: Pupillenmitte
- 33: Sichtkante
- 34: Sichtkante
- 35: Referenzsichtebene
- 36: Nullblickrichtungsebene
- 37: Referenzpunkt
- 38: vertikale Linien

## Patentansprüche

1. Vorrichtung zur Erfassung der Höhe einer Pupillenmitte (32) über dem unteren Rand (31) einer Brillenfassung oder eines Brillenglases, wobei die Vorrichtung zur Anordnung an der Brillenfassung oder dem Brillenglas (2) eines Benutzers ausgebildet ist, mit einer an einer weitgehend horizontalen Drehachse (24) drehbaren ersten Einrichtung (16) zur Bestimmung der benutzerspezifischen Nullblickrichtung während natürlicher Kopf- und Körperhaltung des Benutzers und eine zweite Einrichtung (27, 28) zur Ermittlung der Pupillenmitte des Benutzers auf der Basis der benutzerspezifischen Nullblickrichtung, wobei
die erste Einrichtung ein massebehaftetes Schwenkbauteil (16) ist, das an der horizontalen Drehachse (24) frei drehbar gelagert ist derart, dass eine durch zwei an dem Schwenkbauteil (16) vorgesehene Bezugsstrecken (27, 28) aufgespannte Ebene bei veränderlicher Kopf- und Körperhaltung des Benutzers parallel zur benutzerspezifischen Nullblickrichtung verläuft und wobei die zweite Einrichtung zwei an der ersten Einrichtung im Abstand angeordnete und eine vorbestimmte Länge aufweisende Stifte sind, die gleichzeitig die Bezugsstrecken sind.

2. Vorrichtung nach Anspruch 1, **dadurch gekennzeichnet, dass** das Schwenkbauteil (16) arretierbar ist derart, dass in der während natürlicher Kopf- und Körperhaltung des Benutzers arretierten Stellung die von den Bezugsstrecken (27, 28) aufgespannte Ebene parallel zur benutzerspezifischen Nullblickrichtung verläuft.

3. Vorrichtung nach Anspruch 1 oder 2, **dadurch gekennzeichnet, dass** die Stifte (27, 28) querschnittlich quaderförmig ausgebildet sind und Sichtkanten ausbilden, wobei sich eine durch vorbestimmte Sichtkanten (33, 34) aufgespannte Ebene in einem vorbestimmten Abstand mit einer die Pupillenmitte (32) und die benutzerspezifische Nullblickrichtung enthaltenden Nullblickrichtungsebene (36) schneidet derart, dass sich eine in Höhe der Schnittgerade befindende Pupillenmitte (Op 2) eines Betrachters genau in der Höhe der Pupillenmitte (32) des Benutzers befindet.

4. Vorrichtung nach einem der vorstehenden Ansprüche, **gekennzeichnet durch** einen langgestreckten Träger (5), an dessen Enden jeweils zwei weitgehend vertikal verlaufende Bügel (11, 12) angeordnet sind, mittels derer die Vorrichtung lösbar an der Brillenfassung oder dem Brillenglas (2) anbringbar ist und an dessen axialer Mitte ein gabelförmiger Ausleger (13) angeordnet ist, an dem das Schwenkbauteil (16) drehbar angeordnet ist.

5. Vorrichtung nach Anspruch 4, **dadurch gekennzeichnet, dass** an dem Ausleger (13) ein Arretierbügel (17) verschwenkbar angelenkt ist derart, dass mittels einer Verschwenkbewegung des Arretierbügels (17) das Schwenkbauteil (16) aus seiner frei drehbaren Stellung in die arretierte Stellung überführbar ist.

6. Vorrichtung nach Anspruch 4, **dadurch gekennzeichnet, dass** an einem Bügel (11) eine skalierte Messeinrichtung (29) verschiebbar anbringbar ist derart, dass der Nullpunkt (30) der Messeinrichtung (29) mit dem unteren Rand (31) der Brillenfassung oder des Brillenglases (2) in Überdeckung bringbar ist und der Abstand vom Nullpunkt (30) zur Pupillenmitte (32) mittels der Messeinrichtung (29) bestimmbar ist.

7. Vorrichtung nach Anspruch 5 oder 6, **dadurch gekennzeichnet, dass** an dem Schwenkbauteil (16) eine skalierte Messeinrichtung (25) zur Ermittlung der Vorneigung der Brillenfassung (Fassungsvorneigung) vorgesehen ist derart, dass die Fassungsvorneigung am Schwenkbauteil (16) ablesbar ist.

8. Vorrichtung nach einem der Ansprüche 4 bis 7, **dadurch gekennzeichnet, dass** an dem langgestreckten Träger (5) zwei Referenzpunkte (37) in einem vorbestimmten Abstand angeordnet sind, die eine Referenzskala bilden, mittels der Abmessungen aus einer von dem Benutzer mit Brillenfassung und Vorrichtung (1) aufgenommenen Abbildung mittels elektronischer Bildverarbeitung in skalierte Werte konvertierbar sind und die Höhe der Pupillenmitte (32) über dem unteren Rand (31) der Brillenfassung oder des Brillenglases (2) bestimmbar ist.

9. Vorrichtung nach Anspruch 8, **dadurch gekennzeichnet, dass** an den beiden Armen (14, 15) des gabelförmigen Auslegers (13) jeweils eine vertikale Linie (38) in einer vorbestimmten Relativstellung zu den Referenzpunkten (37) angebracht ist derart, dass zur Vermeidung einer vertikalen Parallaxe mittels elektronischer Bildverarbeitung bestimmbar ist, ob die aufgenommene Abbildung vertikal von vorne oder in einer von der Vertikalen seitlich abweichenden Richtung aufgenommen wurde.

10. Vorrichtung nach einem der vorstehenden Ansprüche zur Verwendung bei der Erfassung des Abstandes der Pupillenmitte über dem unteren Rand einer Brillenfassung oder eines Brillenglases.

## Claims

1. Device for detecting the height of the middle (32) of a pupil above the lower edge (31) of a spectacle frame or a spectacle lens, the device being designed to be located on the spectacle frame or the spectacle lens (2) of a user, with a first arrangement (16) which is pivotable at a largely horizontal pivot axis (24) for determining the user-specific zero viewing direction while the head and body of the user are in a natural attitude, and a second arrangement (27, 28) for determining the middle of the pupil of the user on the basis of the user-specific zero viewing direction, the first arrangement being a weighted swivelling component (16) which is mounted pivotable freely at the horizontal pivot axis (24) such that a plane defined by two reference lines (27, 28) provided on the swivelling component (16) runs parallel to the user-specific zero viewing direction while the attitude of the head and body of the user is changing, and the second arrangement being two pins which are located at a distance on the first arrangement and exhibit a predetermined length and which at the same time form the reference lines.

2. Device according to claim 1, **characterised in that** the swivelling component (16) can be locked such that in the locked position while the head and body of the user are in a natural attitude the plane defined by the reference lines (27, 28) runs parallel to the user-specific zero viewing direction.

3. Device according to claim 1 or 2, **characterised in that** the pins (27, 28) are parallelepiped in cross-section and form viewing edges, and a plane defined by predetermined viewing edges (33, 34) at a predetermined distance intersects with a zero viewing direction plane (36) containing the middle (32) of the pupil and the user-specific zero viewing direction such that a pupil middle (Op 2) of a viewer level with the intersecting straight line is exactly level with the middle (32) of the pupil of the user.

4. Device according to one of the preceding claims, **characterised by** an elongated carrier (5) at the ends of which are arranged in each case two arms (11, 12) running largely vertically by means of which the device can be attached detachably to the spectacle frame or the spectacle lens (2) and at the axial centre of which is arranged a fork-shaped bracket (13) on which the swivelling component (16) is arranged pivotably.

5. Device according to claim 4, **characterised in that** a locking arm (17) is articulated pivotably on the bracket in such a way that the swivelling component (16) can be brought from its freely pivotable position into the locked position by means of a swivelling movement of the locking arm (17).

6. Device according to claim 4, **characterised in that** a scaled measuring arrangement (29) can be attached detachably to an arm (11) in such a way that the zero point (30) of the measuring arrangement (29) can be brought into alignment with the lower edge (31) of the spectacle frame or the spectacle lens (2) and the distance from the zero point (30) to the middle (32) of the pupil can be determined by means of the measuring arrangement (29).

7. Device according to claim 5 or 6, **characterised in that** a scaled measuring arrangement (25) is provided on the swivelling component (16) to determine the inclination of the spectacle frame (inclination of the frame) such that the inclination of the frame can be read off the swivelling component (16).

8. Device according to one of claims 4 to 7, **characterised in that** arranged on the elongated carrier (5) at a predetermined distance apart there are two reference points (37) which form a reference scale by means of which measurements from a picture taken of the user with the spectacle frame and device (1) can be converted into scaled values by means of electronic image processing and the height of the middle (32) of the pupil above the lower edge (31) of the spectacle frame or the spectacle lens (2) can be determined.

9. Device according to claim 8, **characterised in that** in each case a vertical line (38) is formed on the two arms (14, 15) of the fork-shaped bracket (13) in a predetermined relative position to the reference points (37) in such a way that to avoid a vertical parallax it is possible to use electronic image processing to determine whether the recorded picture was taken vertically from the front or in a direction departing laterally from the vertical.

10. Device according to one of the preceding claims for use in determining the distance of the middle of the pupil above the lower edge of a spectacle frame or a spectacle lens.

## Revendications

1. Dispositif pour la détection de l'élévation du centre d'une pupille (32) au-dessus du bord inférieur (31) d'une monture de lunettes ou d'un verre à lunettes, ledit dispositif étant réalisé pour être agencé sur la monture de lunettes ou sur le verre à lunettes (2) d'un utilisateur, comprenant un premier moyen (16) capable de tourner sur un axe de rotation (24) largement horizontal et destiné à déterminer la direction horizontale du regard spécifique à l'utilisateur pendant un maintien naturel de la tête et du corps de l'utilisateur, et un second moyen (27, 28) pour déterminer le centre de la pupille de l'utilisateur en se basant sur la direction horizontale du regard spécifique à l'utilisateur, dans lequel le premier moyen est un composant pivotant (16) chargé d'une masse, lequel est monté en rotation libre sur l'axe de rotation horizontal (24) de telle manière qu'un plan défini par deux droites de référence (27, 28) prévues sur le composant pivotant (27) s'étend parallèlement à la direction horizontale du regard spécifique à l'utilisateur alors que le maintien de la tête et du corps de l'utilisateur se modifie, et le second moyen est constitué par deux tiges agencées à distance sur le premier moyen et présentant une longueur prédéterminée, lesquelles constituent simultanément les droites de référence.

2. Dispositif selon la revendication 1, **caractérisé en ce que** le composant pivotant (16) est susceptible d'être bloqué de telle façon que dans la position bloquée pendant le maintien naturel de la tête et du corps de l'utilisateur, le plan défini par les droites de référence (27, 28) s'étend parallèlement à la direction horizontale du regard spécifique à l'utilisateur.

3. Dispositif selon la revendication 1 ou 2, **caractérisé en ce que** les tiges (27, 28) sont réalisées avec une section transversale de forme carrée et réalisent des arêtes de vision, un plan défini par des arêtes de vision prédéterminées (33, 34) recoupant à une distance prédéterminée un plan de direction horizontale du regard (36) qui contient le centre de la pupille (32) et la direction horizontale du regard, de telle façon que le centre d'une pupille (Op 2) d'un observateur qui se trouve à la hauteur de la droite d'intersection se trouve exactement à l'élévation du centre de la pupille (32) de l'utilisateur.

4. Dispositif selon l'une des revendications précédentes, **caractérisé par** un support allongé (5), aux extrémités duquel sont agencés respectivement deux étriers (11, 12) s'étendant largement verticalement, au moyen desquels le dispositif peut être monté de façon détachable sur la monture de lunettes ou sur le verre à lunettes (2), et au milieu axial du support est agencé un bras (13) en forme de fourche sur lequel le composant pivotant (16) et agencé avec possibilité de rotation.

5. Dispositif selon la revendication 4, **caractérisé en ce qu'**un étrier de blocage (17) est articulé en pivotement sur le bras (13) de telle façon qu'au moyen d'un mouvement de pivotement de l'étrier de blocage (17), le composant pivotant (16) peut être transféré de sa position en rotation libre jusque dans la position bloquée.

6. Dispositif selon la revendication 4, **caractérisé en ce qu'**un dispositif de mesure doté d'une échelle (29) est susceptible d'être monté déplaçable sur un étrier (11), de telle façon que le point zéro (30) du dispositif de mesure (29) peut être amené en coïncidence avec le bord inférieur (31) de la monture de lunettes ou du verre à lunettes (2), et la distance du point zéro (30) jusqu'au centre de la pupille (32) peut être déterminée au moyen du dispositif de mesure (29).

7. Dispositif selon la revendication 5 ou 6, **caractérisé en ce qu'**un dispositif de mesure doté d'une échelle (25) est prévu sur le composant pivotant (16) pour déterminer l'inclinaison négative de la monture de lunettes (inclinaison de monture), de telle façon que l'inclinaison négative de la monture est lisible sur le composant pivotant (16).

8. Dispositif selon l'une des revendications 4 à 7, **caractérisé en ce que** deux points de référence (37) sont agencés à une distance prédéterminée sur le support allongé (5), lesquels forment une échelle de référence au moyen de laquelle des mesures prélevées d'une image de l'utilisateur prise avec monture de lunettes et dispositif (1) peuvent être converties en valeurs échelonnées au moyen d'un traitement d'imagerie électronique, et l'élévation du centre de la pupille (32) au-dessus du bord inférieur (31) de la monture de lunettes ou du verre à lunettes (2) peut être déterminée.

9. Dispositif selon la revendication 8, **caractérisé en ce qu'**une ligne verticale respective (38) est apposée sur les deux branches (14, 15) du bras en forme de fourche (13), dans une position relative prédéterminée par rapport aux points de référence (37), de telle façon que pour éviter une parallaxe verticale, il est possible de déterminer au moyen d'un traitement d'imagerie électronique si l'image prise a été prise verticalement de devant ou dans une direction qui dévie latéralement par rapport à la verticale.

10. Dispositif selon l'une des revendications précédentes destiné à être utilisé pour la détection de la distance du centre de la pupille au-dessus du bord inférieur d'une monture de lunettes ou d'un verre à lunettes.
